(19) Europäisches Patentamt
European Patent Office
Office européen des brevets

(11) Numéro de publication: 0 097 550
B1

(12) **FASCICULE DE BREVET EUROPÉEN**

(45) Date de publication du fascicule du brevet: 15.01.86

(51) Int. Cl.⁴: C 07 C 149/24, A 61 K 31/13

(21) Numéro de dépôt: 83401079.5

(22) Date de dépôt: 30.05.83

(54) **Procédé de préparation de dérivés de la cystamine à activité oncostatique.**

(30) Priorité: 04.06.82 FR 8209767

(43) Date de publication de la demande: 04.01.84 Bulletin 84/1

(45) Mention de la délivrance du brevet: 15.01.86 Bulletin 86/3

(84) Etats contractants désignés: AT BE CH DE GB IT LI LU NL SE

(56) Documents cité: EP-A-0 021 991

(73) Titulaire: Etablissement Public dit: CENTRE NATIONAL DE LA RECHERCHE SCIENTIFIQUE (CNRS), 15, Quai Anatole France, F-75007 Paris (FR)

(72) Inventeur: Oiry, Joel, 31, Lotissement Château Bon Route de Lavérune, F-34100 Montpellier (FR)
Inventeur: Imbach, Jean- Louis, "Clos des Oliviers" 1108 rue de Las Sorbes, F-34000 Montpellier (FR)

(74) Mandataire: Warcoin, Jacques, Cabinet Régimbeau 26, avenue Kléber, F-75116 Paris (FR)

EP 0 097 550 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1986

**Description**

La présente invention concerne un procédé de préparation de dérivés de la cystamine présentant une activité oncostatique.

La demande de brevet français n° 79 15672, déposée le 19 octobre 1979 au nom de l'AGENCE NATIONALE DE VALORISATION DE LA RECNERCHE (ANVAR), décrit notamment de nouveaux composés à activité oncostatique ayant la formule suivante:

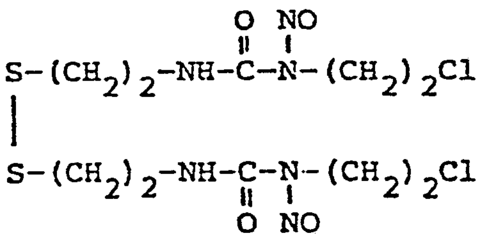

et

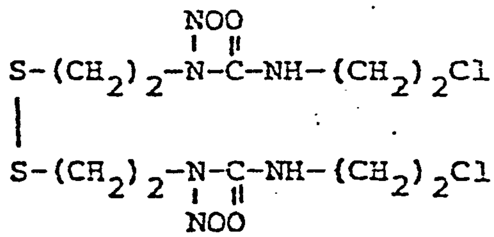

Ces produits sont également actifs en mélange comme cela a été démontré dans les exemples du brevet français mentionné précédemment.

La présente invention concerne un nouveau procédé de préparation de ce type de composés et un procédé particulier permettant de séparer l'un de ces produits qui présente des propriétés particulièrement avantageuses.

Selon le procédé de la présente invention, on prépare les composés de formule:

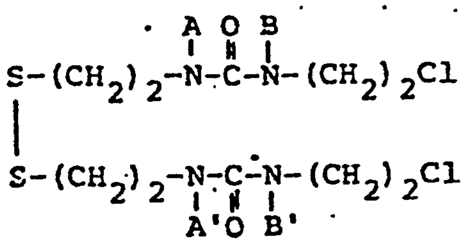

dans laquelle l'un des radicaux A' ou B' et A ou B représente -NO et l'autre représente un atome d'hydrogène, par nitrosation du carbamate de formule I:

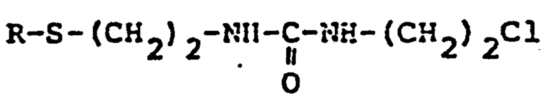

(I)

dans laquelle R est un radical protecteur de la fonction alcool hydrolysable en milieu acide.

Dans le procédé selon la présente invention, la nitrosation est effectuée, de préférence, par un nitrite de métal alcalin dans l'acide formique.

Parmi les radicaux protecteurs de la fonction alcool, il faut citer notamment le radical trityle.

Cette réaction de nitrosation est conduite, de préférence, à une température comprise entre 5 et 20°C.

Le carbamate de formule (I) peut être préparé notamment par action du dérivé de cystéamine de formule II:

$$R-S-(CH_2)_2-NH_2 \qquad (II)$$

sur le chloro-2 éthylisocyanate.

Le produit direct de nitrosation constitué d'un mélange d'isomères est utilisable à titre de médicament pour ses propriétés oncostatiques, au même titre que les mélanges mentionnés dans le brevet français précédent.

Les différents composants du mélange de nitrosation peuvent être séparés par des procédés connus, en particulier la cristallisation fractionnée.

Selon l'invention, par cristallisation fractionnée dans un mélange éther-acétate d'éthyle, on peut isoler l'isomère mixte de formule:

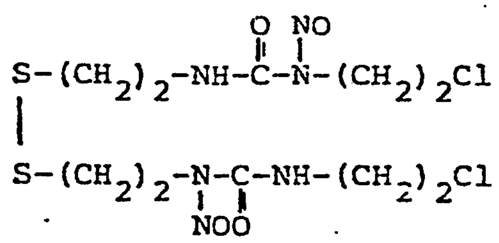

cet isomère n'ayant pu être préparé par synthèse directe.

La presente invention concerne également l'application à titre de médicament des mélanges obtenus par la mise en oeuvre du procédé selon la présente invention ainsi que de l'isomère mixte. Ces différents éléments présentent des propriétés oncostatiques et peuvent être utilisés au même titre et dans des conditions qui ont été décrites dans le brevet français précédent.

D'autres caractéristiques du procédé selon l'invention apparaîtront à la lecture de l'exemple ci-après.

**EXEMPLE**

Procédé de synthèse d'un mélange de divers isomères selon la présente invention

- S-trityl N-chloro-2 éthylcarbamoylcystéamine

Une solution de 100 $cm^3$ d'ether anhydre et de 2,8 g ($88x10^{-4}$ mole) de S-tritylcystéamine est agitée à environ 0°C et additionnée goutte à goutte de 0,77 $cm^3$ ($9x10^{-3}$ mole) d'isocyanate de chloro-2 ethyle. A la fin de l'addition, on laisse le mélange revenir à la température ambiante et on maintient l'agitation durant 12 heures.

Le précipité crème formé est essoré, lavé à l'éther glacé et séché dans un dessicateur sous vide sur anhydride phosphorique. F = 80-85°C. On recueille 3 g de poudre crème qui cristallise du dichlorométhane sous forme d'aiguilles crème. F = 96-97°C.

Analyse: $C_{24}$ $H_{25}$ S $N_2$ 0 Cl (424,5)
calcule (%): C 67,84 H 5,88 N 6,59
trouvé (%): C 67,52 H 5,92 N 6,27

- Obtention de la CNCC par nitrosation de la S-trityl N-chloro-2 éthylcarbamoylcystéamine

On dissout 424,5 mg ($10^{-3}$ mole) du S-tritylcarbamate précédent dans 5 $cm^3$ d'acide formique pur à température ambiante. Il y a de suite une coloration jaune de le solution et formation d'un précipité de triphénylcarbinol.

Le mélange réactionnel est alors amené à environ 10°C et ensuite additionné de 276 mg ($4x10^{-3}$ mole) de nitrite de sodium durant 1 heure. Le précipité de triphénylcarbinol est essoré et les eaux-mères sont additionnées de 50 $cm^3$ d'eau distillée glacée. L'huile orangée formée est extraite par 3 x 50 $cm^3$ d'acétate d'éthyle.

Les phases organiques rassemblées sont lavées à l'eau distillée (2 x 50 $cm^3$) avec une solution saturée glacée de bicarbonate de sodium aqueux (50 $cm^3$) puis à l'eau distillée jusqu'à pH neutre. La solution organique est ensuite séchée sur sulfate de sodium et évaporée à sec sous vide. On détecte en ccm (éluant: éther-hexane 8-2) deux spots visibles à l'U.V., Rf = 0,15 et 0,4.

Les deux produits (rendement global 70 %) sont séparés sur colonne de silice sèche (Kieselgel 60, 70-230 mesch, Merck) avec un éluant constitué d'un mélange d'éther et d'hexane 7-3.

Le premier produit majoritaire (Rf = 0,4) cristallisé dans un mélange d'éther et d'hexane (v/v). Cristaux jaunes F = 77-78°C. Rendement 60 %.

Ce produit comparé à la CNCC est en tous points identique à celle décrite dans le précédent brevet français (Analyse. IR, RMN, IH, HPLC). Par cinq recristallisations successives de cette CNCC, constituée d'un mélange d'isomères, dans de l'éther-acétate d'éthyle (15-1), il est possible d'isoler l'isomère mixte pur.

On obtient alors un composé de formule brute $C_{10}H_{18}S_2N_6O_4Cl_2$ ayant un point de fusion de 83-84°C ayant un spectre infrarouge (KBr)v $cm^{-1}$ de:

(NH) 3325; (CH) 3000, 2960, 2930, 2910; (C=O) 1690; (amide) 1525; (N-NO) 1480;

et un spectre de RMN ($CDCl_3$) de:

$N\underline{H}$ : (m) centré à 7,40 ppm ; 2 H échangeables à $D_2O$

$\underline{CH_2}-\underset{NO}{N}$ : (m) centré à 4,17 ppm ; 4 H

$NH-\underline{CH_2}$ ; $\underline{CH_2}Cl$ (avec NO côté soufre) ; (m) centré à 3,80 ppm ; 6 H

$\underline{CH_2}Cl$ (avec NO côté chlore) : (t) centré à 3,49 ppm ; 2 H

S $\underline{CH_2}$ (NO côté chlore) : (t) centré à 2,99 ppm ; 2 H

S $\underline{CH_2}$ (NO côté soufre) : (m) centré à 2,71 ppm ; 2 H.

Le second produit Rf =0,15 obtenu lors de la nitrosation du S-tritylcarbamate a pu être également isolé par chromatographie sur silice. Son rendement est inférieur à 5%. Il a été identifié par RMN du proton comme étant un dérivé nitrosé de la cystéamine. Spectre de RMN ($CDCl_3$) $\underline{H}S$: (t) 1,15 ppm.

Formule : 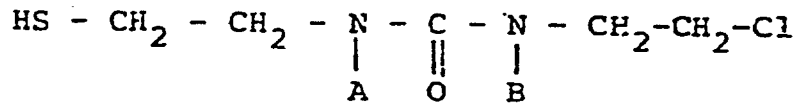

1) A = H, B = NO

2) A = NO, B = H.

Des tests pharmacodynamiques ont permis de déterminer pour l'isomère mixture une DL 50 aiguë par voie intrapéritonéale de 95 mg/kg.

**Revendications**

1) Procédé de préparation des composés de formule:

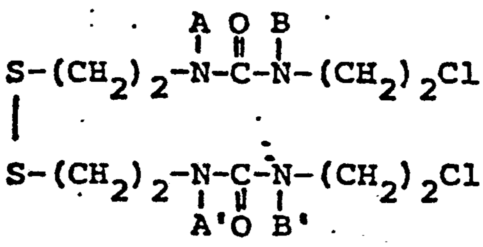

dans laquelle l'un des radicaux A' ou B' et A ou B represente -NO et l'autre représente un atome d'hydrogène, caractérisé en ce qu'on effectue la nitrosation du carbamate de formule I:

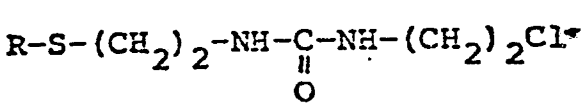 (I)

dans laquelle R est un radical protecteur de la fonction alcool hydrolysable en milieu acide.

2) Procéde selon la revendication 1, caractérisé en ce que la nitrosation est effectuée par le nitrite de sodium dans l'acide formique.

3) Procédé selon l'une des revendications 1 et 2, caractérisé en ce que R est le radical trityle.

4) Procédé selon l'une des revendications 1 à 3, caractérisé en ce que la réaction est conduite entre 5 et 20°C.

5) Procédé selon l'une des revendications 1 à 4, caractérisé en ce que le carbamate de formule I est obtenu par réaction de la chloro-2 éthylisocyanate sur le dérivé de S-cystéamine correspondant.

6) Procédé de préparation du composé de formule:

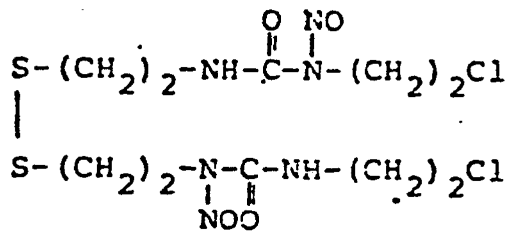

caractérisé en ce qu'on effectue une cristallisation fractionnée du mélange obtenu par la mise en oeuvre du procédé selon l'une des revendications 1 à 5.

**Patentansprüche**

1. Verfahren zur Herstellung von Verbindungen der Formel

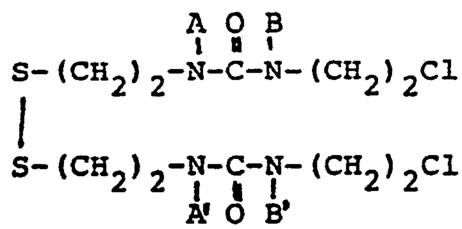

worin einer der Reste A' oder B' und A oder B -NO und der andere ein Wasserstoffatom bedeutet, dadurch gekennzeichnet, daß man die Nitrosierung des Carbamates der Formel

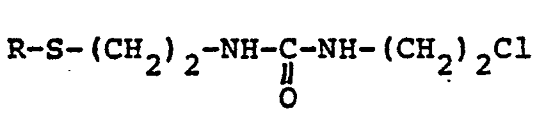 (I),

worin R eine in saurem Milieu hydrolysierbare Schutzgruppe der Alkoholfunktion ist.

2. Verfahren nach Anspruch 1, dadurch gekennzeichnet, daß die Nitrosierung mittels Natriumnitrit in Ameisensäure durchgeführt wird.

3. Verfahren nach einem der Ansprüche 1 und 2, dadurch gekennzeichnet, daß R der Tritylrest ist.

4. Verfahren nach einem der Ansprüche 1 bis 3, dadurch gekennzeichnet, daß die Reaktion zwischen 5 und 20°C ausgeführt wird.

5. Verfahren nach einem der Ansprüche 1 bis 4, dadurch gekennzeichnet, daß das Carbamat der Formel I durch Reaktion von Chlor-2-ethylisocyanat mit dem entsprechenden Derivat von S-Cysteamin erhalten wird.

6. Verfahren zur Herstellung der Verbindung der Formel

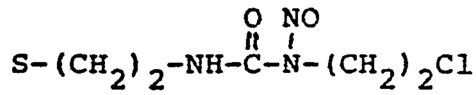

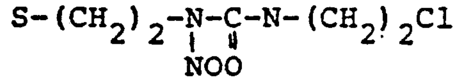
,

dadurch gekennzeichnet, daß man eine fraktionierte Kristallisierung des durch Anwendung des Verfahrens nach einem der Ansprüche 1 bis 5 erhaltenen Gemisches durchführt.

**Claims**

1. Method for the preparation of compounds of formula:

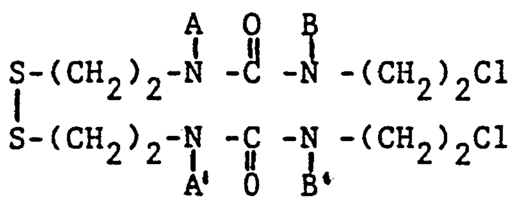

in which one of the radicals A' or B' and A or B represents -NO and the other represents a hydrogen atom, characterised in that one carries out the nitrosation of the carbamate of formula I:

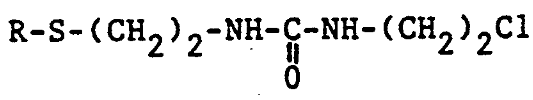
(I)

in which R is a radical protecting the alcohol function and which is hydrolyzable in an acid medium.

2. Method according to Claim 1, characterised in that the nitrosation is effected by sodium nitrite in formic acid.

3. Method according to one of Claims 1 and 2, characterised in that R is the trityl radical.

4. Method according to one of Claims 1 and 3, characterised in that the reaction is carried out between 5 and 20°C.

5. Method according to one of Claims 1 to 4, characterised in that the carbamate of formula I is obtained by reaction of the 2-chloro ethylisocyanate with the corresponding derivative of S-cysteamine.

6. Method for the preparation of the compound of formula:

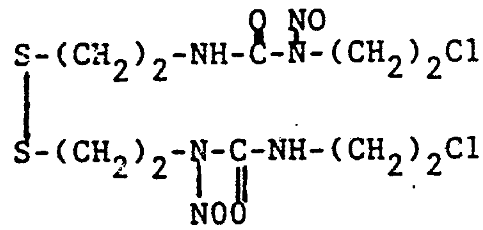

characterised in that one carries out a fractionated crystallisation of the mixture obtained by implementing the method according to one of Claims 1 to 5.